# EUROPÄISCHE PATENTSCHRIFT

(11) **EP 2 129 311 B1**
(45) Veröffentlichungstag und Bekanntmachung des Hinweises auf die Patenterteilung: **18.04.2012**
(21) Anmeldenummer: 08734691.2
(22) Anmeldetag: 20.03.2008
(51) Int. Cl.: A61B 17/88, A61B 17/00, A61B 17/16

(54) **MANIPULATOR FÜR RUNDMATERIAL, INSBESONDERE FÜR EINEN KIRSCHNERDRAHT**
MANIPULATOR FOR ROUND MATERIAL, PARTICULARLY FOR A KIRSCHNER WIRE
MANIPULATEUR POUR MATÉRIAUX RONDS, EN PARTICULIER POUR UNE BROCHE DE KIRSCHNER

(30) Priorität: 24.03.2007 DE 102007014730
(43) Veröffentlichungstag der Anmeldung: 09.12.2009
(73) Patentinhaber: Hörmansdörfer, Gerd, 31303 Burgdorf-Beinhorn (DE)
(72) Erfinder: Hörmansdörfer, Gerd, 31303 Burgdorf-Beinhorn (DE)
(74) Vertreter: Siekmann, Gunnar
(86) Internationale Anmeldenummer: PCT/EP2008/002254
(87) Internationale Veröffentlichungsnummer: WO 2008/116600

(56) Entgegenhaltungen:
- EP-A- 1 410 863
- GB-A- 1 257 777
- US-A- 2 468 946
- US-A1- 2003 216 738
- US-B1- 6 673 078

## Beschreibung

Die Erfindung betrifft einen Manipulator für dünnes Rundmaterial und Draht, insbesondere für einen so genannten Kirschnerdraht. Bei einem Kirschnerdraht - auch Spickdraht genannt - handelt es sich um einen Metalldraht, welcher als chirurgisches Hilfsmittel Verwendung findet, und mit dem z. B. Knochenfragmente stabilisiert und fixiert werden können. Er wird ferner als Pilotdraht für die Vorbereitung bestimmter chirurgischer Eingriffe benutzt. Im allgemeinen besitzen Kirschnerdrähte Durchmesser zwischen etwa 0,6 und 3,5 Millimeter. Der Kirschnerdraht ist nach dem Chirurgen Martin Kirschner (1879-1942) benannt.

Da ein Kirschnerdraht selbst glatt und ohne Ansätze beschaffen ist, wird zu dessen Handhabung ein Manipulator benötigt, mit welchem er erfaßt und bewegt werden kann. Bei bisherigen Manipulatoren kommt es vor, daß die von Hand mittels eine Drehbewegung aufzubringende Klemmkraft zu keiner absolut sicheren Fixierung des Drahtes führt. Wenn der Draht dann auf einen Widerstand stößt, kann er durch den Klemmmechanismus des Manipulators rutschen und dabei nach Durchstoßen des Schutzhandschuhs die Hand des Chirurgen verletzen. Außerdem wäre dann das weitere Vorschieben des Drahtes nur mit Schwierigkeiten zu bewerkstelligen. Im ersten Fall wäre die erforderliche Sterilität der Instrumente nicht mehr gewährleistet und ferner der Chirurg von der Übertragung ansteckender Krankheiten des Patienten bedraht.

Bei bislang bekannten Manipulatoren besteht außerdem die Gefahr, daß die rigiden Spannmittel derartig dünne Drähte beim Klemmvorgang beschädigen und es auf Grund solcher Beschädigungen beim Manipulieren zum Drahtbruch kommen kann. Ein Fischen des abgebrochenen Drahtendes ist dann nur mit Schwierigkeiten zu bewerkstelligen.

Aus der GB 1 257 777 (Basis für den Oberbegriff des Anspruchs 1) ist ein Werkzeughalter bekannt, welcher für die in der Lange verstellbare Aufnahme runder Werkzeuge, wie z. B. Bohrer oder Fräser, vorgesehen ist. Für die eigentliche Spannung des Werkzeugs wird die Heranziehung eines Differenzialgewindes, auch bezeichnet als Differenzgewinde, vorgeschlagen. Entsprechend dem Anwendungsbereich - es dürfte sich bei den Werkzeugen um solche mit einem Schaftdurchmesser zwischen etwa 8 und 20 Millimeter handeln - ist dieser Werkzeughalter relativ robust ausgelegt. Da die eigentliche Spannzange außerdem mit einem ihrer beiden Gewinde selbst Teil des Differentialgewindes ist, kann eine Spannvorrichtung der beschriebenen Art kaum Vorbild für einen Manipulator für dünnen Runddraht sein. Insbesondere für die Verwendung eines Spannmittels aus einen Elastomer, ist das in der GB 1 257 777 gezeigte Konzept nicht geeignet.

Es bestand daher die Aufgabe zur Schaffung eines Manipulators für dünnes Rundmaterial und Draht, insbesondere für einen Kirschnerdraht, welcher die genannten Probleme überwinden und eine absolut sichere und schonende Klemmung ohne die Gefahr des Drahtbruches garantieren sollte. Daneben war ein möglichst einfacher Bauaufwand mit einer Herstellbarkeit zu moderaten Kosten gefordert.

Nach der Erfindung wird die beschriebene Aufgabe durch Schaffung eines neuartigen Manipulators gemäß Patentanspruch 1 sowie den verschiedenen Unteransprüchen gelöst. Danach wird vorgeschlagen, in den Mechanismus des Manipulators eine dem Anwendungsbereich angepaßte spezielle Bauform eines Differenzialgewindes zu integrieren.

Differenzialgewinde sind im Prinzip bekannt. Differenzialgewinde bestehen aus drei Schraubpartnern mit insgesamt zwei Gewindepaaren gleichen Windungssinns, wobei die beiden Gewindepaare jedoch unterschiedliche Steigungen besitzen. Eines der Gewinde des ersten Gewindepaares ist gegenüber einem Gewinde des zweiten Gewindepaares sowohl gegen Verschiebung längs der Achse als auch gegen Verdrehung gesichert. Das andere Gewinde des ersten Gewindepaares ist gegenüber dem anderen Gewinde des zweiten Gewindepaares gegen Verdrehung gesichert, nicht jedoch gegen Verschiebung längs der Achse. Bei einer gegenseitigen Verdrehung der einen Gewinde zu den anderen Gewinden verschieben sich diese relativ zueinander lediglich mit der Differenz ihrer Steigungen. Dadurch lassen sich mit einem Differenzialgewinde hohe axiale Verspannungskräfte realisieren.

Die Erfindung soll im folgenden anhand von fünf Zeichnungsfiguren näher erläutert werden. Drei der Zeichnungen zeigen Ausführungsbeispiele eines Manipulators für einen Kirschnerdraht in einer maßstabsgerechten Größe von 1:1. Dabei wurde eine partiell geschnittene Darstellung gewählt, um vor allem die innen liegenden Details sichtbar zu machen. In der Zeichnungsfigur 4 ist die innen liegende Hülse und in Zeichnungsfigur 5 eine Spannzangenkolonne dargestellt, beide zwecks besserer Erkennbarkeit im Maßstab von 1,5: 1, davon letztere als Schnitt.

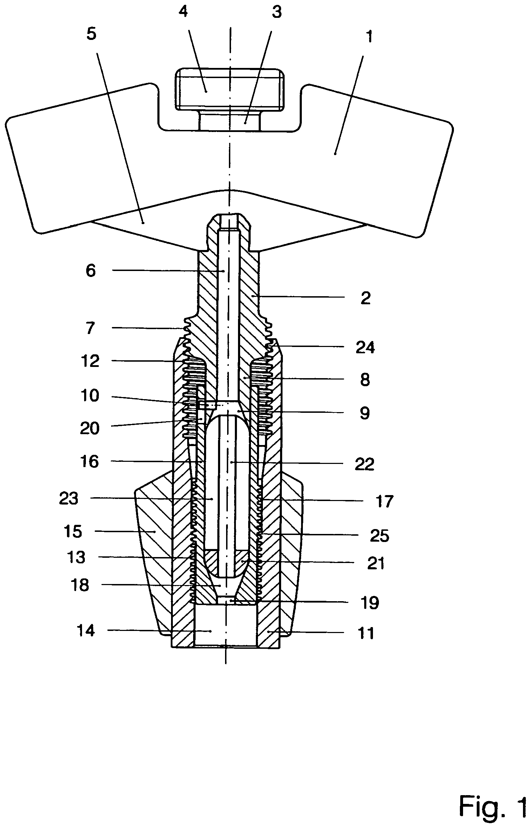

Fig. 1 zeigt einen erfindungsgemäßen Manipulator mit einer zentralen Welle 2 aus Metall, welcher hinter einem angespritzten Handstück 1 aus einem Elastomer nach einem Bund 3 in einer Art Vierkant 4 endet. Zum Handstück gehört ferner ein Ansatz 5, ebenfalls aus einem Elastomer. Die Welle weist eine zentrale Bohrung 6 auf, welche noch unten hin in einen Konus 9 übergeht. Die Welle ist in einem Teilbereich mit einem Außengewinde 7 der Abmessung Tr 20 x 2 versehen. Danach geht sie in eine Verjüngung 8 über. Zum Manipulator gehört ferner eine Doppelmutter 11, welcher mit einer Ummantelung 15 aus einem Elastomer umspritzt ist. Zwecks Erzielung einer besseren Griffigkeit zeigt diese Ummantelung in der Radialebene eine Kontur in Form eines dreiflügeligen Polygons. Die Doppelmutter ist oben mit einem gröberen Innengewinde. 12 der Abmessung Tr 20 x 2 und unten mit einem feineren Innengewinde 13 der Abmessung Tr 17 x 1,5 ausgestattet. Das untere Innengewinde läuft nach unten hin in einer zylindrischen Bohrung 14 aus. Wahlweise kann es auch bis zum unteren Ende der Doppelmutter geschnitten sein. Innerhalb der Doppelmutter ist eine Hülse 16 positioniert. Diese ist mit einem Außengewinde 17 der Abmessung Tr 17 x 1,5 ausgerüstet. Der Zeichnungsfigur ist zu entnehmen, daß die Verjüngung 8 der Welle 2 in das obere Ende der Hülse 16 eintaucht. Die Hülse 16 ist mit einem Schlitz 20 versehen. Dieser kann in den unterschiedlichsten Formen realisiert sein, z. B. als Langloch oder in Gestalt eines "J". Ein in der Welle verankerter Stift 10 greift in den Schlitz ein und verhindert so eine Relativverdrehung zwischen der Welle 2 und der Hülse 16. In der Zeichnungsfigur wird nur ein Schlitz und nur ein Stift gezeigt. Es ist unter Umständen günstiger, wenigstens zwei solcher Verdrehsicherungen vorzusehen. Die Hülse 16 weist eine durchgehend zylindrische Bohrung auf, welche sich nach ihrem unteren Ende zu über einen Konus 18 auf eine Durchlaßbohrung 19 verjüngt. Zwischen dem Konus 9 der Welle 2 und dem Konus 18 der Hülse 16 ist eine Spannzange 21 gelagert. Schlitze 23 beliebiger Art, so lange sie geeignet sind, der Spannzange eine erforderliche Elastizität zu verleihen, sind vorhanden. Die zentrale Bohrung 22 der Spannzange ist in ihrem Durchmesser auf den zu klemmenden Runddraht oder Kirschnerdraht so abgestimmt, daß ein Durchschieben des zu klemmenden Elements im entspannten Zustand und eine ausreichende Klemmung im gespannten Zustand problemlos möglich ist.

Die Doppelmutter ist im gezeigten Beispiel mit Überlappungen 24, 25 von lediglich jeweils etwa 2 Umdrehungen mit Welle und Hülse verschraubt, wobei diese Positionen dem gelösten Zustand des Manipulators entsprechen. Beim Verdrehen der Doppelmutter 11 gegen Welle 2 und Hülse 16 kommt es bei dem gezeigten Beispiel aufgrund der Steigungsdifferenz der beteiligten Gewinde zu einer relativen axialen Verschiebung zwischen Welle und Hülse von 0,5 mm pro Umdrehung. Wenn nun die Verdrehung im Uhrzeigersinn erfolgt, dann wird der gegenseitige Abstand zwischen Welle und Hülse kleiner. Die beiden Konusflächen 9 und 18 bewegen sich demgemäß aufeinander zu und üben auf die kugelkuppenartigen Endflächen der Spannzange einen entsprechenden Druck aus, welcher zu einer Verkleinerung sowohl des äußeren als auch des inneren Durchmessers der Spannzange führt. Dadurch und durch die hohe Untersetzung des Systems wird eine sichere kraftschlüssige Spannung des zu klemmenden Elementes garantiert.

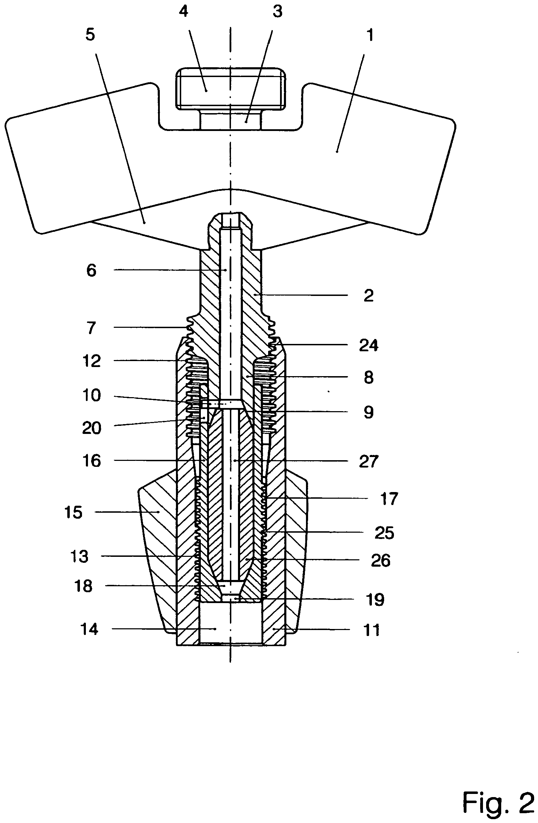

In Fig. 2 wird der Manipulator aus Fig. 1 mit identischen Bezugszahlen gezeigt. Lediglich die Spannzange aus Metall wurde gegen eine mit einer Bohrung 27 versehene Spannbuchse 26 aus einem Elastomer, z. B. Polyurethan, getauscht. Die Spannbuchse weist keine Schlitze auf. Sie taucht beiderseits in die konischen Spannflächen 9 und 18 ein. Die in der Zeichnung gewählte Eintauchtiefe hat zeichnerische Gründe. Bei einer praktischen Ausführung wird die Eintauchtiefe möglicherweise tiefer in die Konusflächen von Welle 2 und Hülse 16 eintauchen. Wird nun auf die Spannbuchse ein axialer Druck ausgeübt, so verhält sich diese wie eine nicht komprimierbare Flüssigkeit und bekneift mit den Wandungen ihrer Bohrung 27 das kraftschlüssig zu verklemmende Element. Diese Art der Verspannung ist wegen der gleichmäßigen Kraftverteilung äußerst schonend, sodaß Beschädigungen des zu verklemmenden Elementes ausgeschlossen sind. Außerdem können damit sehr kleine Durchmesser von z. B. kleiner als 1 Millimeter problemlos gespannt werden.

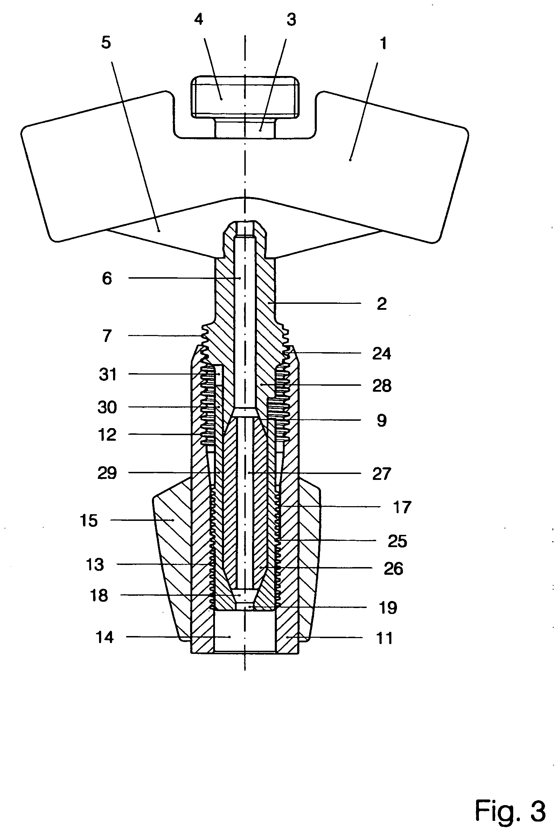

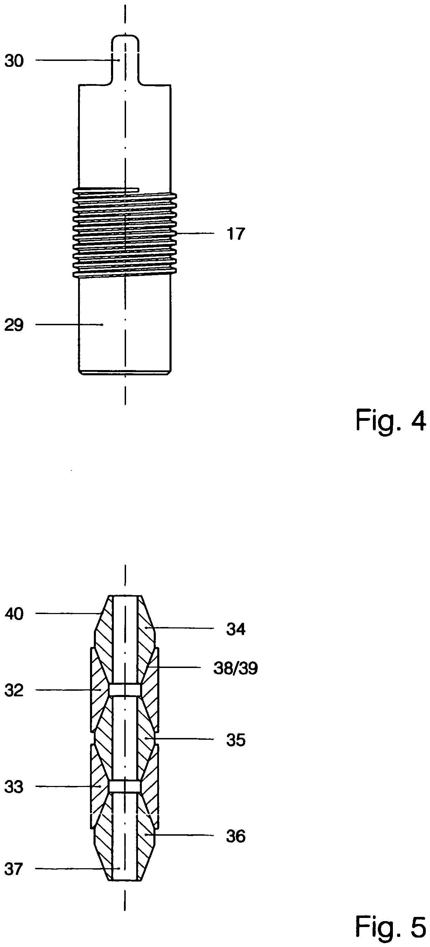

Das in Fig. 3 dargestellte Ausführungsbeispiel eines erfindungsgemäßen Manipulators entspricht weitestgehend demjenigen aus Fig. 2. Entsprechend wurden sämtliche Gleichteile und selben Details mit unveränderten Bezugszahlen versehen. Es wird nun lediglich eine andere Art der Verdrehsicherung gezeigt. Diese besteht aus einer modifizierten, abgestuften Verjüngung 28 mit einer eingefrästen Längsnut 31, sowie einer modifizierten Hülse 29, welche jetzt mit einer axial herausstehenden Zunge 30 gestaltet ist. Die mit ihrer eigenen Breite mit der Breite der Längsnut korrespondierende Zunge laucht in die Längsnut ein und kann sich darin in Längsrichtung verschieben, ohne daß dadurch eine gegenseitige Verdrehung zwischen Welle 2 und Hülse 16 stattfinden kann. Diese Art der Gestaltung ist sehr rigide und außerdem sehr einfach per Fräsen herstellbar, wobei auch z. B. zwei oder drei derartige Verdrehsicherungen realisierbar sind. Zum besseren Verständnis der konstruktiven Details der Erfindung ist in Fig. 4 die Außenansicht der Hülse 29 aus Fig. 3 in einem etwa 1:1,5-fach vergrößerten Verhältnis dargestellt. Es ist hier das etwa mittig angeordnete Gewinde 17 (Tr 17 x 1,5) und die axial herausstehende Zunge 30 zu sehen.

Eine spezielle Ausführung der Spannzange in Form einer Spannfederkolonne wird in Fig. 5 mittels einer geschnitten und im Maßstab von etwa 1,5:1 vergrößerten Darstellung gezeigt. Die Spannfederkolonne wird aus zwei äußeren Ringfedern 32, 33 und drei inneren Ringfedern 34, 35, 36 gebildet. Die inneren Ringfedern besitzen eine durchgehende Bohrung 37. Die äußeren Ringfedern korrespondieren mit ihrem Außendurchmesser mit dem Innendurchmesser der Hülse 16 oder 29. Bei extrem kleinen zu klemmenden Drähten empfiehlt sich die Verwendung einer Adapterhülse zwischen der Hülse und der Spannfederkolonne. Dadurch können die Ringfedern deutlich dünner und damit elastischer werden. Sämtliche Ringfedern sind in Längsrichtung mit einem besonders schmalen Schlitz ausgestattet (nicht dargestellt), um unter dem Einfluß einer axialen Spannkraft eine Durchmesseranderung zu erlauben. Dabei gleiten die einzelnen Ringfedern mit ihren gegenseitigen Konusflächen 38/39 gegeneinander. Für den hier gezeigten seitlichen Konuswinkel 40 wurde aus zeichnerischen Gründen ein Wert von 20° gewählt. Jedoch ist dieser Wert nicht bindend. Es wird allerdings empfohlen, für den Konuswinkel einen Wert zu realisieren, welcher nicht zu einer schwer lösbaren Selbsthemmung führt.

Die Erfindung wurde anhand des obigen Textes und der Zeichnungsfiguren sehr detailliert beschrieben. Tatsächlich kann die praktische Verkörperung jedoch sehr unterschiedlich ausfallen. Dies beginnt bereits mit der Wahl des zu klemmenden Durchmessers und des angemessenen Differenzialgewindes (Metrisches ISO-Regelgewinde oder Zollgewinde, Trapezgewinde, Grob- oder Feingewinde, Differenz der Steigung) und betrifft ferner das Einsatzgebiet und z. B. die zu verwendenden Werkstoffe. Beispielsweise besteht bei einer Anwendung im Bereich der Medizintechnik die Forderung nach einer Sterilisierbarkeit. Es dürfen dann ausschließlich nichtrostende Werkstoffe zur Anwendung kommen. Wenn dann keine Schmiermittel verwendet werden dürfen, muß der Manipulator auch im Trockenlauf ohne Störung funktionieren. Um in diesem Fall ein Fressen des Gewindes mit absoluter Sicherheit zu unterbinden, wird weiter vorgeschlagen, bei den Gewindepaaren jeweils einen Partner aus nichtrostendem Edelstahl, bzw. so genanntern Chirurgenstahl, oder anderen Werkstoffen dieser Gruppe heranzuziehen, und den jeweils anderen aus Aluminium-Mehrstoffbronze herzustellen.

Mit der Erfindung wird somit ein Manipulator für dünnes Rundmaterial und Draht, insbesondere für Kirschnerdraht, zur Verfügung gestellt, welcher selbst bei einer reinen Betätigung von Hand drastisch erhöhte Klemmkräfte gewährleistet. Trotzdem werden keine Spannmarken auch bei sehr dünnen Drähten erzeugt, wodurch die Gefahr des Bruches durch Kerbwirkung beseitigt ist. Die Handhabung durch den Chirurgen ist sicher, weil das lästige Durchrutschen bekannter Manipulatoren der Vergangenheit angehört. Der Manipulator arbeitet auch im Trockenlauf ohne Störung. Er ist mit moderatem Aufwand zu günstigen Kosten herstellbar.

## Patentansprüche

1. Manipulator mit einem Handgriff und einem mit dem Handgriff verbundenen Mechonismus zum Klemmen von dünnem Rundmaterial und Draht, insbesondere für Spick- oder Kirschnerdraht, wobei der Mechanismus das Ensemble eines Differenzialgewindes aus Doppelmutter (11), Hülse (16, 29) und Welle (2) umfaßt, die Doppelmutter zwei Gewinde (12,13) unterschiedlicher Steigung besitzt, Hülse (16,29) und Welle (2) mit ihrem jeweiligen Außengewinde (17,7) im Inneren der Doppelmutter gelagert sind und das Außengewinde der Hülse mit dem Gewinde kleinerer Steigung der Doppelmutter und das Außengewinde der Welle mit dem Gewinde größerer Steigung der Doppelmutter korrespondieren, **dadurch gekennzeichnet, daß** die Welle (2) in die Hülse (16,29) partiell eintaucht und der so gebildete, mindestens partiell zylindrische Hohlraum zur Aufnahme eines Spannmittels (26) sich zu beiden Enden des Hohlraums hin auf eine jeweilige Durchgangsbohrung verjüngt.

2. Manipulator gemäß Anspruch 1, **dadurch gekennzeichnet, daß** Mittel, zur Unterbindung einer relativen Verdrehung der Hülse (16,29) gegenüber der Welle (2) vorhanden sind.

3. Manipulator gemäß Anspruch 2, **dadurch gekennzeichnet, daß** die Mittel aus mindestens einem Stift (10) an einem der Partner und mindestens einem axialen Schlitz (20) oder Langloch am anderen Partner gebildet sind.

4. Manipulator gemäß Anspruch 2, **dadurch gekennzeichnet, daß** die Mittel aus mindestens einem Stift (10) an einem der Partner und mindestens einem Schlitz (20) in Form eines "J" oder eines unvollkommenen "U" am anderen Partner gebildet sind.

5. Manipulator gemäß Anspruch 2, **dadurch gekennzeichnet, daß** die Mittel aus mindestens einer Zunge (30) an einem der Partner und mindestens einer axialen Nut am anderen Partner gebildet sind.

6. Manipulator gemäß einem oder mehreren der Ansprüche 1 bis 5, **dadurch gekennzeichnet, daß** zwischen Welle (2) und Hülse (16,29) eine Spannmittel (26) vorhanden ist, dessen Innendurchmesser mit dem Durchmesser des zu spannenden Rundmaterials, Drahtes oder Kirschnerdrahtes korrespondiert.

7. Manipulator gemäß Anspruch 6, **dadurch gekennzeichnet, daß** das Spannmittel (26) aus einer geschlitzten Spannzange besteht.

8. Manipulator gemäß Anspruch 6, **dadurch gekennzeichnet, daß** das Spannmittel (26) aus einer nachgiebigen Spannbuchse besteht, welche aus einem Elastomer, z. B. Polyurethan, hergestellt ist.

9. Manipulator gemäß Anspruch 6, **dadurch gekennzeichnet, daß** das Spannmitel (26) aus einer Kolonne von Ringfedern gebildet ist.

10. Manipulator gemäß Anspruch 9, **dadurch gekennzeichnet, daß** die Ringfedern in axialer Richtung geschlitzt sind.

11. Manipulator gemäß einem oder mehreren der Ansprüche 1 bis 10, **dadurch gekennzeichnet, daß** bei den Gewindepaarungen jeweils eines der Gewinde aus der Gruppe der nichtrostenden Edelstähle, und das andere aus einer Aluminium-Mehrstoffbronze gefertigt ist.

## Claims

1. A manipulator having a handle and a mechanism which is connected to the handle and serves for clamping thin round material and wire, in particular a pin or Kirschner wire, wherein the mechanism comprises the ensemble of a differential thread consisting of a double nut (11), a sleeve (16, 29) and a shaft (2), the double nut has two threads (12, 13) with different pitches, the sleeve (16, 29) and the shaft (2) are mounted with their respective external thread (17, 7) inside the double nut, and the external thread of the sleeve corresponds with the double nut's thread having a smaller pitch and the external thread of the shaft corresponds with the double nut's thread having a larger pitch, **characterized in that** the shaft (2) partially immerses into the sleeve (16, 29) and the at least partially cylindrical hollow space formed in this manner tapers toward both ends of the hollow space to form a respective through-hole for receiving a clamping means (26).

2. The manipulator according to claim 1, **characterized in that** means for preventing a relative rotation of the sleeve (16, 29) with respect to the shaft (2) are provided.

3. The manipulator according to claim 2, **characterized in that** said means are formed from at least one pin (10) on one of the partners and at least one axial slot (20) or oblong hole on the other partner.

4. The manipulator according to claim 2, **characterized in that** said means are formed from of at least one pin (10) on one of the partners and at least one slot (20) in the form of a "J" or an incomplete "U" on the other partner.

5. The manipulator according to claim 2, **characterized in that** said means are formed from at least one tongue (30) on one of the partners and at least one axial groove on the other partner.

6. The manipulator according to one or more of the claims 1 to 5, **characterized in that** between the shaft (2) and the sleeve (16, 29), a clamping means is provided, the inner diameter of which corresponds with the diameter of the round material, wire, or Kirschner wire to be clamped.

7. The manipulator according to claim 6, **characterized in that** the clamping means (26) consists of a slotted collet.

8. The manipulator according to claim 6, **characterized in that** said clamping means (26) consists of a resilient clamping sleeve which is made from an elastomer, e.g. polyurethane.

9. The manipulator according to claim 6, **characterized in that** said clamping means (26) is formed from a column of annular springs.

10. The manipulator according to claim 9, **characterized in that** the annular springs are slotted in the axial direction.

11. The manipulator according to one or more of the claims 1 to 10, **characterized in that** in the case of the mating threads in each case one of the threads is made of the group of stainless steels and the other one is made of an aluminum multi-alloy bronze.

## Revendications

1. Manipulateur comprenant une manette et un mécanisme relié à la manette pour serrer du matériau rond mince et du fil, en particulier pour du fil à broche ou du fil de Kirschner, le mécanisme comprenant l'ensemble d'un filetage différentiel constitué d'un double écrou (11), d'une gaine (16, 29) et d'un arbre (2), le double écrou présentant deux filetages (12, 13) de pente différente, la gaine (16, 29) et l'arbre (2) étant montés avec leur filetage extérieur (17, 7) respectif à l'intérieur du double écrou et le filetage extérieur de la gaine correspondant au filetage de pente plus petite du double écrou et le filetage extérieur de l'arbre au filetage de pente plus grande du double écrou, **caractérisé en ce que** l'arbre (2) plonge partiellement dans la gaine (16, 29) et la cavité ainsi formée, au moins partiellement cylindrique, destinée au logement d'un moyen de serrage (26), se rétrécit en direction des deux extrémités de la cavité pour former un alésage de passage respectif.

2. Manipulateur selon la revendication 1, **caractérisé en ce que** des moyens pour supprimer une torsion relative de la gaine (16, 29) par rapport à l'arbre (2) sont présents.

3. Manipulateur selon la revendication 2, **caractérisé en ce que** les moyens constitués d'une broche (10) sont formés sur l'un des partenaires et au moins une fente axiale (20) ou un trou oblong axial sur l'autre partenaire.

4. Manipulateur selon la revendication 2, **caractérisé en ce que** les moyens constitués d'au moins une broche (10) sont formés sur l'un des partenaires et au moins une fente (20) sous forme d'un "J" ou d'un "U" incomplet sur l'autre partenaire.

5. Manipulateur selon la revendication 2, **caractérisé en ce que** les moyens sont constitués d'au moins une lame (30) sur l'un des partenaires et d'au moins une rainure axiale sur l'autre partenaire.

6. Manipulateur selon l'une ou plusieurs des revendications 1 à 5, **caractérisé en ce qu'**un moyen de serrage (26), dont le diamètre intérieur correspond au diamètre du matériau rond, du fil ou du fil de Kirschner à serrer, est présent entre l'arbre (2) et la gaine (16, 29).

7. Manipulateur selon la revendication 6, **caractérisé en ce que** le moyen de serrage (26) est constitué d'une pince de serrage fendue.

8. Manipulateur selon la revendication 6, **caractérisé en ce que** le moyen de serrage (26) comprend une douille de serrage flexible, qui est fabriquée à base d'un élastomère, par exemple du polyuréthane.

9. Manipulateur selon la revendication 6, **caractérisé en ce que** le moyen de serrage (26) est formé d'une colonne de ressorts annulaires.

10. Manipulateur selon la revendication 9, **caractérisé en ce que** les ressorts à bague sont fendus dans le sens axial.

11. Manipulateur selon l'une ou plusieurs des revendications 1 à 10, **caractérisé en ce que**, dans les accouplements de filetage, à chaque fois l'un des filetages est fabriqué à partir du groupe des aciers fins inoxydables et l'autre à base d'un bronze multi-produits d'aluminium.
